# EUROPÄISCHE PATENTANMELDUNG

(11) **EP 1 394 246 A1**
(43) Veröffentlichungstag der Anmeldung: **03.03.2004**
(21) Anmeldenummer: 02013653.7
(22) Anmeldetag: 20.06.2002
(51) Int. Cl.: C12M 1/107, B01F 7/16

(54) **Rühreinrichtung für einen Fermenter einer Biogasanlage sowie Verfahren zur Verteilung von Biomasse in einer Fermenterflüssigkeit mit einer Rühreinrichtung**

(71) Anmelder: U.T.S. Umwelt-Technik-Süd GmbH, 84419 Obertaufkirchen (DE)
(72) Erfinder: Bürger, Adam, 84405 Grüntegernbach (DE)
(74) Vertreter: Liebl, Thomas, Dipl.-Ing.

(57) **Zusammenfassung**

Die Erfindung betrifft eine Rühreinrichtung (1) für einen Fermenter (2) einer Biogasanlage sowie ein Verfahren zur Verteilung von Biomasse (7) in einer Fermenterflüssigkeit (8) mit einer Rühreinrichtung (1). Erfindungsgemäß sind zwei einander zugeordnete im Betriebszustand gegensinnig angetriebene Rührerwellen (10, 11) als Rührerwellenpaar (9) vorgesehen, die in etwa parallel zueinander ausgerichtet sind und die sowohl eine lokale, im Wesentlichen turbulente Rührwellenströmung (18) um die jeweilige Rührerwelle (10, 11) herum für eine intensive lokale Vermischung der Biomasse (7) mit der Fermenterflüssigkeit (8) und ferner eine im Wesentlichen horizontale, gerichtete Umwälzströmung (19) im gesamten Volumen des Fermenterbehälters (3) ausbilden. Dadurch wird eine besonders gute Durchmischung und Verteilung der Biomasse (7) in der gesamten Fermenterflüssigkeit (8) erzielt.

## Beschreibung

Die Erfindung betrifft eine Rühreinrichtung für einen Fermenter einer Biogasanlage nach dem Oberbegriff des Anspruchs 1 und ein Verfahren zur Verteilung von Biomasse in einer Fermenterflüssigkeit mit einer Rühreinrichtung nach dem Oberbegriff des Anspruchs 21.

In Biogasanlagen läuft ein Fermentationsprozess ab, bei dem organische Stoffe, wie beispielsweise Wirtschaftsdünger aus der Landwirtschaft (Rindergülle, Rinder-Festmist, Schweinegülle, Schweine-Festmist, Hühnergülle, Hühner-Trockenkot) und/oder landwirtschaftliche Reststoffe (Grasschnitt-Rübenblätter, Silagen) und/oder Reststoffe aus der Agroindustrie oder verwandten Industrien (Biertreber, Obstreste, Gemüsereste, Rapsschrot, Getreideabputz, Schlempen, Melasse) als Biomasse vergast werden. Die hierbei entstehenden Gase sammeln sich in einem oberen Fermenterbehälterbereich eines Fermenterbehälters und können direkt zur Energieerzeugung verwendet werden, z. B. als Heizgas zur Stromerzeugung in nachgeschalteten Brennkraftmaschinen mit Elektrogeneratoren. Zur Fermentation werden im Fermenterbehälter die organischen Stoffe mit Flüssigkeit versetzt und Mikroorganismen, wie z. B. Hefen, Bakterien, etc. zugeführt, so dass der Fermentations- bzw. der Vergasungsprozess unter aeroben oder anaeroben Bedingungen ablaufen kann.

Ein Problem bei derartigen allgemein bekannten Fermentationsprozessen ist, dass die Biomasse, insbesondere Biomasse-Feststoffe in der Fermenterflüssigkeit regelmäßig nicht gleichmäßig verteilt sind, da sie z. B. aufschwimmen und sich im Bereich der Flüssigkeitsoberfläche ansammeln, was z. B. der Fall ist, wenn das spezifische Gewicht der Biomasse geringer ist als dasjenige der Fermenterflüssigkeit, z. B. Wasser. Andererseits kann aber auch das Problem bestehen, dass Biomasse, insbesondere Biomasse-Feststoffe mit einem höheren spezifischen Gewicht als die Fermenterflüssigkeit auf den Fermenterbehälterboden absinken und sich dort als Sinkschichten ansammeln. Auch eine derartige Ansammlung von Biomasse bewirkt eine ungleichmäßige Biomasseverteilung in der Fermenterflüssigkeit, was sich insgesamt nachteilig auf den Wirkungsgrad des Fermentationsprozesses auswirkt.

Für eine Verteilung der Biomasse in der Fermenterflüssigkeit sind bereits Tauchmotorrührgeräte als Rühreinrichtungen bekannt, die mittels einer Seilwinde entlang eines Aggregatträgers verfahrbar sind. So ist aus der gattungsgemäßen DE 197 32 168 C1 eine Rühreinrichtung für einen Fermenter einer Biogasanlage mit einer Rührerwelle bekannt, die wenigstens einen Rührer aufweist, wobei die Rührerwelle mittels einem Rührerwellenantrieb antreibbar ist und wobei die Rührerwelle in eine in einem Fermenterbehälterinnenraum aufnehmbare und mit Biomasse versetzte Fermenterflüssigkeit eintauchbar ist zum Umwälzen der Fermenterflüssigkeit zur Verteilung der Biomasse in der Fermenterflüssigkeit.

Konkret ist die Rühreinrichtung hier durch ein Tauchmotorrührgerät gebildet, das an einem vertikal im Behälter angeordneten Aggregatträger höhenverstellbar gehalten ist. Eine Seiltrommel ist hier innerhalb im oberen Bereich des Fermenterbehälters angeordnet, so dass keine gasdichte Seildurchführung durch eine Fermenterbehälterwand erforderlich ist. Eine außerhalb des Behälters angeordnete Betätigungseinrichtung für den Seiltrommelantrieb ist mit dem mittels einer dichten Durchführung durch eine Behälterwand geführten Seiltrommelantrieb verbunden. Das Tauchmotorrührgerät wird hier durch einen Tauchmotor gebildet, der eine herkömmliche Rührerwelle mit einem Propellerrührer antreibt. Eine derartige Rühreinrichtung mit einem an einer Rührerwelle angeordneten Propellerrührer führt nur zu einer Vermischung der Biomasse und der Fermenterflüssigkeit in einem lokal begrenzten Bereich innerhalb der Fermenterflüssigkeit. Um in unterschiedlichen Fermenterflüssigkeitsbereichen eine Durchmischung zu erreichen, ist das Tauchmotorrührgerät mittels dem Aggregatträger um dessen Längsachse zu verschwenken, damit die Rührerwelle mitsamt Propellerrührer auch andere bezüglich der Biomasseansammlungen kritische Fermenterflüssigkeitsbereiche durchmischt. Hierzu sind insbesondere im Hinblick auf die Seiltrommelanordnung komplizierte Getriebemechaniken und/oder Gelenkanordnungen erforderlich, um das Verschwenken des Tauchmotorrührgerätes um den Aggregatträger zu ermöglichen. Jedoch ergibt sich auch hier wiederum das Problem, dass auch im verschwenkten Zustand dann nur eine Vermischung in diesem begrenzten Bereich innerhalb der Fermenterflüssigkeit einstellt. Insbesondere in rührerwellenfernen Fermenterflüssigkeitsbereichen ergibt sich regelmäßig eine Entmischung zwischen der Biomasse und der Fermenterflüssigkeit, d. h. es kommt zu einer unerwünschten Phasentrennung und damit zur erneuten Ausbildung von unerwünschten Schwimm- oder Sinkschichten. Dies beruht insbesondere auch darauf, dass die Abmessungen der Fermenterbehälter, insbesondere deren Innendurchmesser regelmäßig sehr groß gegenüber den Abmessungen der Rührerwellen mitsamt Propellerrührer ist. So liegen beispielsweise die Fermenterbehälterdurchmesser zwischen 13-15 m, während eine Rührerwelle relativ klein, z. B. im Bereich von 0,5 m, ausgebildet ist. Insgesamt wird somit trotz des Einsatzes derartiger Tauchmotorrührgeräte aufgrund der nach wie vor gegebenen ungleichmäßigen Biomasseverteilung bezogen auf die gesamte Fermenterflüssigkeit der Wirkungsgrad des Fermentationsprozesses nur unwesentlich erhöht.

Aus Explosionsschutzgründen ist zudem die Anwesenheit von elektrischen Leitungen im eine Gasphase aufweisenden Fermenterbehälterinnenraum problematisch, da dies aus sicherheitstechnischen Gründen eine dauerhafte, zeitaufwendige Kontrolle der Anlage erfordert.

Aufgabe der Erfindung ist es daher, eine Rühreinrichtung für einen Fermenter einer Biogasanlage sowie ein Verfahren zur Verteilung von Biomasse in einer Fermenterflüssigkeit mit einer Rühreinrichtung zu schaffen, die bei relativ einfachem Aufbau eine möglichst gleichmäßige Verteilung der Biomasse in der gesamten Fermenterflüssigkeit zur Vermeidung von Biomasse-Schwimmschichten und/oder Biomasse-Sinkschichten und daher eine Erhöhung des Wirkungsgrades des gesamten Fermentationsprozesses ermöglichen.

Diese Aufgabe wird bezüglich der Rühreinrichtung gelöst mit den Merkmalen des Anspruchs 1.

Gemäß Anspruch 1 sind zwei einander zugeordnete und im Betriebszustand gegensinnig angetriebene Rührerwellen als Rührerwellenpaar vorgesehen, die in etwa parallel zueinander ausgerichtet sind und die im in die Fermenterflüssigkeit eingetauchten, angetriebenen Zustand eine lokale, im Wesentlichen turbulente Rührerwellenströmung um die jeweilige Rührerwelle herum ausbilden für eine intensive lokale Vermischung der Biomasse mit der Fermenterflüssigkeit im rührerwellenpaarnahen Fermenterflüssigkeitsbereich. Ferner bilden die in die Fermenterflüssigkeit eingetauchten Rührerwellen im angetriebenen Zustand eine im Wesentlichen horizontale, gerichtete Umwälzströmung im gesamten Volumen des Fermenterbehälters aus dergestalt, dass die im Wesentlichen horizontale Umwälzströmung nach Durchströmen eines Durchströmbereiches zwischen den beiden Rührerwellen von einem im Bereich der Rührerwellen ausgebildeten Überdruckbereich ausgehend in den rührerwellenpaarfernen Fermenterflüssigkeitsbereich wegströmt und anschließend wieder im Kreislauf zu einem Unterdruckbereich als Ansaugbereich im Bereich der Rührerwellen zurückströmt.

Vorteilhaft wird mit einem derartigen Aufbau der Rühreinrichtung mit wenigstens einem Rührerwellenpaar eine sehr intensive lokale Durchmischung der Biomasse mit der Fermenterflüssigkeit im rührerwellenpaarnahen Flüssigkeitsbereich erzielt, so dass die Biomasse in diesem lokalen Bereich in optimaler Weise besonders gleichmäßig in der Fermenterflüssigkeit verteilt ist, wie dies für einen hohen Wirkungsgrad des Fermentationsprozesses insgesamt wünschenswert ist. Durch die im Wesentlichen überlagerte horizontale, gerichtete Umwälzströmung wird hier vorteilhaft zusätzlich die gleichmäßige Verteilung der Biomasse im gesamten Restvolumen des Fermenterbehälters erreicht, d. h. auch in dem rührerwellenpaarfernen Fermenterflüssigkeitsbereich erzielt, da die im Bereich der Rührerwellen durchmischten Fermenterflüssigkeitsbereiche mittels der Umwälzströmung weiterverteilt werden, um die möglichst gleichmäßige Verteilung der Biomasse im gesamten Volumen des Fermenterbehälters zu erzielen.

D. h., dass für den Fall, dass sich Sinkschichten und/oder Schwimmschichten in der Fermenterflüssigkeit ausbilden, die z. B. durch Biomasse-Feststoffe gebildet sind, somit durch die lokale und im Wesentlichen turbulente Rührerwellenströmung eine sehr gute und gleichmäßige Verteilung der einzelnen Biomasse-Feststoffe im gesamten rührerwellenpaarnahen Fermenterflüssigkeitsbereich erzielt wird, d. h., dass die Sinkschichten und/oder Schwimmschichten im Wesentlichen aufgelöst werden. Um auch in dem rührerwellenpaarfernen Fermenterflüssigkeitsbereich eine möglichst gleichmäßige Verteilung der Biomasse-Feststoffe in der Fermenterflüssigkeit zu erzielen, wird mit den Rührerwellen gleichzeitig die im Wesentlichen horizontale, gerichtete Umwälzströmung im gesamten Volumen des Fermenterbehälters ausgebildet, wodurch ausgehend vom lokalen Rührerwellenbereich die eine gleichmäßige Biomasse-Verteilung aufweisenden Fermenterflüssigkeitsteile sozusagen mit der Umwälzströmung auf die Reise geschickt werden, wobei durch die im Kreislauf geführte Umwälzströmung die gleichmäßige Verteilung der Biomasse in der gesamten Fermenterflüssigkeit sichergestellt wird. Sobald es im Verlauf des Umwälzströmungsweges zu einer gewissen Entmischung zwischen der Biomasse und der Fermenterflüssigkeit kommt, werden diese vom Ansaugbereich des Rührerwellenpaares wieder angesaugt und im Bereich der Rührerwellen wieder in der zuvor beschriebenen Art und Weise vermischt, bevor sie wieder in Umwälzströmungsrichtung mit sehr gleichmäßiger Verteilung weiterbefördert werden. Dadurch können Sinkschichten und/oder Schwimmschichten aus Biomasse, insbesondere aus Biomasse-Feststoffen in Fermenterbehältern vorteilhaft sehr gut vermieden werden. Dies insbesondere im Hinblick auf Biogasanlagen, bei denen der Fermenterbehälterdurchmesser in der Größenordnung von 10-20 m, z. B. in der Größenordnung von 13-15 m, liegt und die Rührerwellenanordnung z. B. eine Breite von 1-2 m aufweist, d. h. also der Fermenterbehälter sehr groß gegenüber den Rührerwellen ist.

Mit den Merkmalen der Ansprüche 2 und 3 werden Rühreinrichtungen beansprucht, mit denen vorteilhaft unterschiedliche Arten von Umwälzströmungen in der Fermenterflüssigkeit eingestellt werden können, z. B. eine Umwälzströmung, die sich nach dem Wegströmen vom Überdruckbereich in zwei entgegengesetzte horizontale Umwälzteilströmungen aufteilt. Ebenso ist aber auch eine Anordnung der Rührerwellen als Rührerpaar dergestalt möglich, dass sich im Wesentlichen eine einzige gleichgerichtete Haupt-Umwälzströmung in der Fermenterbehälterflüssigkeit einstellt. Mit derartigen Aufbauten der Rührereinrichtung kann eine vorteilhafte und einfache Anpassung der jeweils benötigten Umwälzströmung an die jeweils gegebenen Fermentationsverhältnisse für eine optimale Verteilung der Biomasse in der Fermenterflüssigkeit gewählt werden. Insbesondere mit einer in etwa senkrecht im Fermenterbehälter angeordneten Mittelsäule, die regelmäßig eine Abstütz- und Tragfunktion für eine Behälterdecke, z. B. eine Behälterbetondecke, übernimmt, kann bei einer exzentrischen Anordnung des Rührerwellenpaars eine vorteilhafte Umwälzströmung in Radialrichtung um die Mittelsäule herum für eine besonders vorteilhafte gleichmäßige Verteilung der Biomasse, insbesondere von Biomasse-Feststoffen in der Fermenterflüssigkeit erzielt werden.

Nach Anspruch 4 können dabei die Rührerwellen grundsätzlich bezogen auf den Fermenterbehälter in etwa senkrecht oder waagrecht ausgerichtet in die Fermenterbehälterflüssigkeit eingetaucht werden. Diese erhöht die Flexibilität beim Einsatz der Rührerwellen als vorteilhafte Rührereinrichtung zur Ausbildung erwünschter Strömungsverhältnisse.

Insbesondere für den Fall, dass die Fermenterbehälterdurchmesser sehr groß gegenüber den Rührerwellenabmessungen und/oder im Verlauf des Umwälzströmungsweges zwischen dem Überdruckbereich und dem Ansaugbereich eine zu große Entmischung zwischen Biomasse und Fermenterflüssigkeit stattfinden würde, kann gemäß Anspruch 5 vorgesehen sein, dass in Umwälzströmungsrichtung gesehen beabstandet zum ersten Rührerwellenpaar wenigstens ein weiteres Rührerwellenpaar vorgesehen ist. Ein derartiger Aufbau ist besonders bevorzugt in Verbindung mit großen Fermenterbehälterdurchmessern, die regelmäßig auch eine Mittelsäule aufweisen, bei der ein erstes exzentrisches Rührerwellenpaar bezogen auf die in etwa senkrechte Mittelsäule in etwa gegenüberliegend zu einem zweiten exzentrischen Rührerwellenpaar angeordnet ist. Die Rührerwellenpaare werden hier jeweils so angetrieben, dass die Umwälzströmung beim Durchströmen des Durchströmbereichs des jeweiligen Rührerwellenpaares nach einer dortigen lokalen intensiven Durchmischung wieder zur Verteilung der Biomasse in der gesamten Fermenterflüssigkeit in Umwälzströmungsrichtung weiterförderbar ist. Je nach Größe und konkretem Aufbau des Fermenterbehälters können ggf. aber auch mehr als zwei Rührerwellenpaare vorgesehen sein. Ein Kriterium für den Einsatz mehrerer Rührerwellenpaare ist somit insbesondere der jeweilige zwischen den einzelnen Rührerwellenpaaren zurückgelegte Umwälzströmungsweg und der jeweilige Grad der Entmischung der Biomasse mit der Fermenterflüssigkeit.

Insbesondere zur Veränderung und/oder Beeinflussung der Umwälzströmungsrichtung kann nach Anspruch 6 vorgesehen sein, dass das Rührerwellenpaar so im Fermenterbehälterinnenraum lagerbar ist, dass dieses um eine in Rührerwellenlängsrichtung verlaufende Schwenkachse verschwenkbar sind. Dabei ist vorzugsweise eine Festlegung in unterschiedlichen Schwenkpositionen vorgesehen.

Nach Anspruch 7 ist für ein Rührerwellenpaar ein gemeinsamer Antrieb für beide Rührerwellen vorgesehen, wobei der Antrieb eine der Rührerwellen antreibt und ferner Mittel vorgesehen sind, mit denen eine Antriebskraft auf die jeweils andere Rührerwelle übertragbar ist. Derartige Mittel können z. B. ein Zahnriemen in Verbindung mit einer Zahnradanordnung zur Drehrichtungsumkehr zur Ausbildung einer gegensinnigen Antriebsrichtung der Rührerwellen eines Rührerpaares sein. Grundsätzlich besteht jedoch auch die Möglichkeit, dass für jede Rührerwelle eines Rührerwellenpaars ein separater Antrieb vorgesehen wird. Ferner können die Rührerwellen je nach gewünschter Richtung der Umwälzströmung in eine Vorwärts- und/oder Rückwärtsrichtung jeweils gegensinnig angetrieben werden bzw. können auch die einzelnen Rührerwellen eines Rührerwellenpaars in Verbindung mit einem gegensinnigen Antrieb unterschiedliche Antriebsgeschwindigkeiten aufweisen, was z. B. bei unterschiedlich ausgebildeten Rührerwellen erforderlich sein kann. Dies ist durch eine entsprechende Umsetzung oder Übersetzung auch bei einem einzigen gemeinsamen Antrieb für beide Rührerwellen besonders einfach realisierbar. Damit ist eine optimale Anpassung an die jeweils konkret gegebenen Bedingungen in Verbindung mit einer Biogasanlage und einem Fermenterbehälter möglich.

Grundsätzlich kann der Antrieb, z. B. ein Elektromotor mit variabler Drehzahlsteuerung, nach Anspruch 8 außerhalb des Fermenterbehälters angeordnet sein. Hierzu ist z. B. dann lediglich ein Antriebsstrang der Rührerwelle dicht durch eine Behälterwand zu führen, z. B. durch eine obere Behälterwand als Behälterdecke, was einfach zu bewerkstelligen ist. Besonders bevorzugt ist jedoch ein Aufbau gemäß Anspruch 9, bei dem der wenigstens eine Antrieb im Fermenterbehälter angeordnet und mittels einem Arbeitsmedium antreibbar ist. Dieser mittels einem Arbeitsmedium antreibbare Antrieb ist mit einer Energieleitung verbunden, die einerseits eine Rohr- und/oder Schlauchleitung als Zuführleitung für das Medium aufweist, die an einer Medium-Kompressionseinheit angeschlossen ist und die dicht durch eine Behälterwand zum Antrieb geführt ist. Weiter umfasst die Energieleitung eine Rohr- und/oder Schlauchleitung als Abführleitung für das Medium, die dicht durch eine Behälterwand aus dem Fermenterbehälter herausgeführt ist. Die Medium-Kompressionseinheit kann dann vorzugsweise elektrisch betrieben und außerhalb des Fermenterbehälters angeordnet werden.

Gemäß einer ersten konkreten Ausführungsform nach Anspruch 10 kann der Antrieb ein Hydraulik-Motor sein, wobei das Medium eine Hydraulikflüssigkeit und die Kompressionseinheit ein Hydraulik-Kompressor ist. Die Abführleitung ist dabei als Rückleitung in einem geschlossenen Kreislauf an den Hydraulik-Kompressor angeschlossen. Alternativ dazu kann nach Anspruch 11 der Antrieb auch durch einen Pneumatik-Motor gebildet sein, wobei als Arbeitsmedium hier dann Luft verwendet ist und die Kompressionseinheit als Luftkompressor ausgebildet ist. Bei dieser Anordnung reicht es aus, wenn die Abführleitung als Abgasleitung lediglich dicht aus dem Fermenterbehälter geführt ist. Bei Verwendung eines anderen Gases, beispielsweise eines Inertgases, kann auch hier ein geschlossener Gaskreislauf mit einer Gasrückführung zum Luftkompressor eingesetzt werden. Sowohl in der Hydraulik- als auch in der Pneumatikausführung ergeben sich die Vorteile, dass die elektrisch betriebenen Kompressoren außerhalb des Fermenterbehälters angeordnet sind und somit der insbesondere aufgrund der Gasphase erforderliche Explosionsschutz die Verwendung von Hydraulik- oder Gasleitungen sowie die entsprechenden Hydraulik- oder Gasmotoren einfach beherrschbar ist. Gegenüber einer elektrischen Energieversorgung mit Elektrokabeln ist zumindest im Fermenterbehälter ein Zwei-Leitungssystem mit Rohr- und/oder Schlauchleitungen als Medium-Zuführleitung und Medium-Abführleitung erforderlich. Insbesondere bei Verwendung eines Pneumatik-Motors ist ein sonst übliches Abblasen von Luft unmittelbar am Motor nicht möglich, da diese Luft in den Flüssigkeitsbereich und dann in den Bereich der Gasphase gelangen würde und dort zu einer unerwünschten Verdünnung des Biogases und einer Gasvermehrung führen würde.

Die Merkmale des Anspruchs 12 wurden bereits zuvor in Verbindung mit den Merkmalen des Anspruchs 7 gewürdigt, so dass hier nicht mehr näher darauf eingegangen wird.

Nach Anspruch 13 ist der Durchströmbereich zwischen den Rührerwellen eines Rührerwellenpaares so ausgebildet, dass die Rührerwellen mitsamt ihrer Rührer entweder einen Spaltabstand voneinander aufweisen oder dass die Rührerwellen mitsamt ihrer Rührer im Betrieb wenigstens teilweise ineinandergreifen. Dies hängt von der jeweiligen konkreten Ausgestaltung der Rührerwellen ab ebenso wie von den Bedingungen im Fermenterbehälter, z. B. den Durchmessergrößen oder aber auch den Anforderungen an den Ansaugbereich und den Überdruckbereich.

Grundsätzlich können die Rührerwellen mit beiden Rührerwellenenden im Bereich der Behälterwände eines Fermenterbehälters gelagert werden, wodurch sich insbesondere eine stabile und sichere Anordnung der Rührerwellen im Fermenterbehälter im montierten Zustand ergibt. Gemäß einer besonderen Ausführungsform nach Anspruch 14 können die Rührerwellen jedoch auch lediglich mit einem Rührerwellenende im Bereich der Behälterwand mittelbar oder unmittelbar gelagert sein, so dass das andere gegenüberliegende Rührerwellenende freitragend, d. h. nicht gelagert ist. Ein derartiger Aufbau kann z. B. dann von Vorteil sein, wenn eine gewisse Ausweichbarkeit, d. h. Verlagerung der Rührerwellen insbesondere im Hinblick auf durch den Durchströmbereich hindurchgepresste große sperrige Biomasse-Feststoffe erforderlich ist, da hierdurch dann die Funktionsfähigkeit weiterhin erhalten wird ohne eine gegebenenfalls erfolgende Beschädigung der Rührerwellenanordnung.

Gemäß einer besonders bevorzugten Ausgestaltung nach Anspruch 15 ist das Rührerwellenpaar jeweils als in den Fermenterbehälter einsetzbares und herausnehmbares Rührerwellenmodul ausgebildet. Besonders bevorzugt weist dabei das Rührerwellenmodul nach Anspruch 16 eine in die Behälterwand einsetzbare Lagerplatte auf, an der die Rührerwellen mit einem Rührerwellenende mittelbar oder unmittelbar gelagert sind. Vorzugsweise ist auch der wenigstens eine Antrieb Bestandteil des Rührerwellenmoduls und je nach Ausführungsart des Antriebs bezogen auf den montierten Zustand des Rührerwellenmoduls wenigstens zum Teil oberhalb oder unterhalb an der Lagerplatte angeordnet. So kann beispielsweise bei einem Hydraulik- oder Pneumatikmotor der Antrieb an der Unterseite der Lagerplatte angeordnet sein, während ein Elektromotor aus Explosionsschutzgründen auf der Oberseite der Lagerplatte montiert sein müsste. Ein derartiges Modul kann z. B. mittels einem Hebezeug, z. B. einem Kran, auf einfache Weise komplett fertig montiert als Vormontagemodul in einen Fermenterbehälter eingesetzt werden, so dass hierdurch die Montagearbeiten auf der Biogasanlagenbaustelle selbst reduziert werden können. Zudem ist ein derartiger Aufbau mit dem Hebezeug auch auf besonders einfache Weise sehr schnell herausnehmbar, z. B. im Falle einer erforderlichen Wartung und/oder Reparatur an einem Modulbestandteil. Ein derartiges Modul kann besonders einfach und schnell im jeweiligen Fertigungsbetrieb vormontiert werden, was grundsätzlich für eine Vielzahl von Modulen wünschenswert ist, da sich hierdurch ein größerer Grad der Automatisierung ergibt.

Nach Anspruch 17 ist im Strömungsbereich der Umwälzströmung wenigstens ein Strömungsleitblech, vorzugsweise zum Leiten der Strömung in Richtung zum Ansaugbereich des Rührerwellenpaars hin, und/oder wenigstens ein Strömungsbrecher, vorzugweise zum Ausbilden einer Vertikalströmungskomponente, vorgesehen. Dadurch lässt sich die jeweils gewünschte Umwälzströmung je nach den individuellen Erfordernissen und Wünschen auf einfache und preiswerte Weise einstellen.

Gemäß einer besonders bevorzugten Ausgestaltung nach Anspruch 18 ist wenigstens eine der Rührerwellen durch eine Schnecke mit Schneckenwendel als Rührer gegebenenfalls zur Ausbildung einer zusätzlichen, überlagerten Vertikalteilströmung gebildet. Die Schnecke kann dabei als seelenlose Schnecke oder aber auch als Bandschnecke ausgebildet sein. Insbesondere für den nach Anspruch 19 bevorzugten Anwendungsfall, dass die Schneckenwendel der Rührerwellen jedes Rührerwellenpaars in einer Doppelfunktion wenigstens teilweise als Zerkleinerungseinrichtung für die in den Durchströmbereich gelangende Biomasse ausgebildet ist, können z. B. an den Schneckenwendelaußenseiten auch Schlagkanten, z. B. durch Aufschweißungen, vorgesehen sein. Eine derartige Doppelfunktion ist besonders vorteilhaft, da hierdurch auch größere Biomasse-Feststoffe, z. B. Äste, Zweige oder dergleichen vorteilhaft zerkleinert werden können, was sich insgesamt positiv auf den Wirkungsgrad des Fermentationsprozesses auswirkt. Die die Rührer bildenden Schneckenwendel können dabei je nach Antriebsrichtung der jeweiligen durch eine Schnecke gebildeten Rührerwelle eine zusätzliche Vertikalströmungskomponente ausbilden, was sich wiederum besonders vorteilhaft auf die gleichmäßige Verteilung der einzelnen z. B. Biomasse-Feststoffe in der gesamten Fermenterflüssigkeit zur Vermeidung von Sinkschichten und/oder Schwimmschichten auswirkt.

Alternativ dazu ist nach Anspruch 20 vorgesehen, dass wenigstens eine der Rührerwellen dort angebrachte, vorzugsweise dort angeschweißte Paddel und/oder Balken und/oder Scheiben und/oder Propeller und/oder Gitter als Rührer aufweist, die gegebenenfalls auch so angestellt sein können, dass sich eine zusätzliche, überlagerte Vertikalströmungskomponente ausbildet. Beispielsweise können die einzelnen Rührerelemente hierzu schräg angestellt werden.

Die Erfindung wird bezüglich des Verfahrens mit den Merkmalen des Anspruchs 21 gelöst.

Die sich hierdurch ergebenden Vorteile und dieselben wie sie bereits zuvor in Verbindung mit der Rühreinrichtung erläutert wurden.

Die Erfindung wird nachfolgend anhand einer Zeichnung näher erläutert.

Es zeigen:
- Fig. 1: einen schematischen Querschnitt als Prinzipskizze durch eine erfindungsgemäße Rühreinrichtung für einen Fermenter einer Biogasanlage,
- Fig. 2: einen schematischen Schnitt entlang der Linie A-A der Fig. 1,
- Fig. 3: einen schematischen Querschnitt durch eine alternative Ausführungsform einer erfindungsgemäßen Rühreinrichtung für einen Fermenter einer Biogasanlage,
- Fig. 4: einen schematischen Schnitt entlang der Linie B-B der Fig. 3,
- Fig. 5: einen schematischen Querschnitt durch eine weitere alternative Ausführungsform einer erfindungsgemäßen Rühreinrichtung für einen Fermenter einer Biogasanlage,
- Fig. 6: eine schematische Schnittansicht entlang der Linie C-C der Fig. 5,
- Fig. 7: einen schematischen Querschnitt durch einen Fermenterbehälter mit verschwenkbarem Rührerwellenpaar,
- Fig. 8: einen schematischen Querschnitt durch eine erfindungsgemäße Rühreinrichtung für einen Fermenter einer Biogasanlage, bei der die Rühreinrichtung als Modul ausgebildet ist, im montierten Zustand,
- Fig. 9: einen schematischen Querschnitt entsprechend Fig. 8 mit teilweise herausgenommenem Modul,
- Fig. 10: einen schematischen Querschnitt entlang der Linie Z-Z1 der Fig. 4 zur beispielhaften Darstellung des Verteilungsgrades von Biomasse in der Fermenterflüssigkeit,
- Fig. 11: einen schematischen Querschnitt entlang der Linie Z-Z2 der Fig. 4 zur beispielhaften Darstellung des Verteilungsgrades von Biomasse in der Fermenterflüssigkeit, und
- Fig. 12: einen schematischen Querschnitt entlang der Linie Z-Z3 der Fig. 4 zur beispielhaften Darstellung des Verteilungsgrades von Biomasse in der Fermenterflüssigkeit.

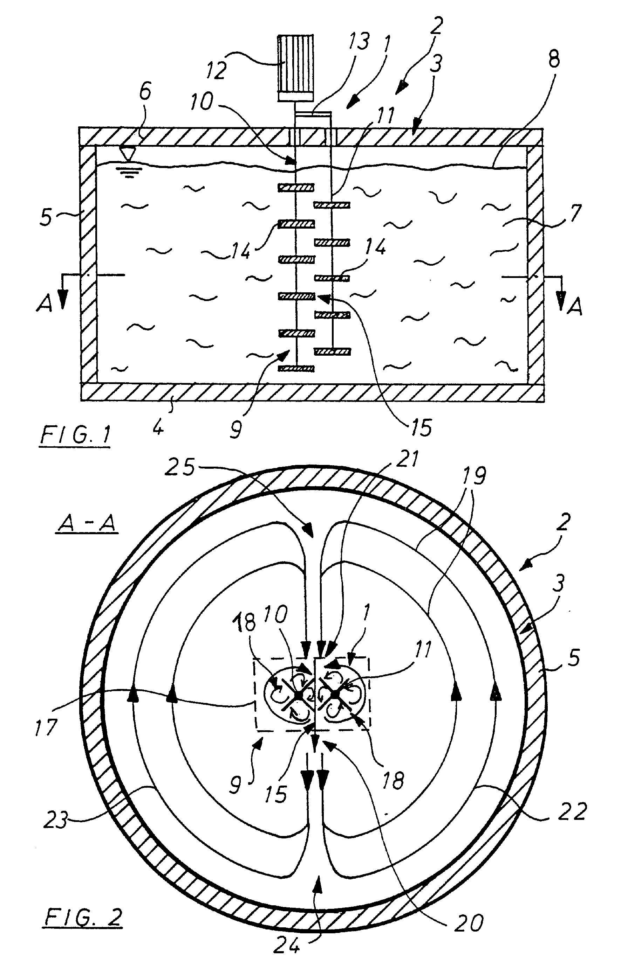

In Fig. 1 ist schematisch ein Querschnitt durch eine erste Ausführungsform einer erfindungsgemäßen Rühreinrichtung 1 für einen Fermenter 2 einer Biogasanlage im Querschnitt gezeigt, während die Fig. 2 einen Schnitt entlang der Linie A-A der Fig. 1 darstellt. Der Fermenter 2 umfasst einen zylindrischen Fermenterbehälter 3, der eine Bodenwand 4, eine Seitenwand 5 und eine Deckenwand 6 aufweist. Im Fermenterbehälter 3 ist eine hier beispielsweise mit Biomasse-Feststoffen 7 versetzte Fermenterflüssigkeit 8 aufgenommen.

Grundsätzlich könnte die Biomasse auch durch ein mehr flüssigkeitsähnliches Material gebildet sein. Ebenso könnte der Fermenterbehälter auch einen wenigstens zum Teil eckigen Querschnitt aufweisen, z. B. einen viereckigen Querschnitt.

Die Rühreinrichtung 1 umfasst ferner ein Rührerwellenpaar 9 aus zwei einander zugeordneten und im Betriebszustand gegensinnig angetriebenen Rührerwellen 10, 11, die parallel zueinander ausgerichtet sind. Für das Rührerwellenpaar 9 ist ein gemeinsamer Antrieb 12, z. B. ein Elektromotor, vorgesehen, der die Rührerwelle 10 antreibt. Um die Antriebskraft auf die zweite Rührerwelle 11 so zu übertragen, dass diese gegensinnig zur Rührerwelle 10 angetrieben wird, sind entsprechende Mittel 13 vorzusehen, z. B. eine Art Keilriemen in Verbindung mit einer Zahnradanordnung. Wie dies insbesondere aus der Fig. 2 ersichtlich ist, ist die Rührerwelle 10 hier in Uhrzeigerrichtung und die Rührerwelle 11 entgegen die Uhrzeigerrichtung angetrieben. Sämtliche Durchführungen durch eine der Behälterwände sind gasdicht auszuführen.

Die beiden Rührerwellen sind hier jeweils lediglich mit einem oberen Rührerwellenende im Bereich der Bodenwand 4 gelagert. Mit ihrem gegenüberliegenden Rührerwellenende sind sie freitragend ausgebildet.

An den Rührerwellen 10, 11 selbst sind hier lediglich äußerst schematisch dargestellte Rührer 14 angeordnet, die z. B. Paddel sein können. Zwischen den beiden Rührerwellen 10, 11 ist ein Durchströmbereich 15 vorgesehen. Dieser Durchströmbereich 15 ist hier so ausgebildet, dass die Rührerwellen 10, 11 im Betrieb in etwa ineinandergreifen, wie dies insbesondere aus der schematischen Darstellung der Fig. 1 zu entnehmen ist.

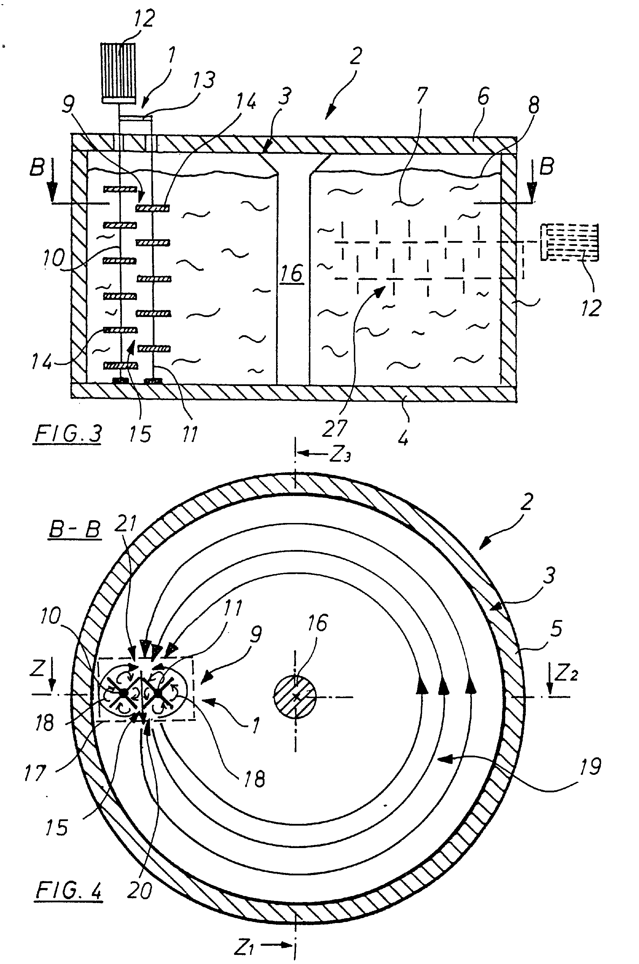

Das Rührerwellenpaar 9 ist hier in einem mittleren, zentralen Fermenterbehälterbereich in etwa senkrecht ausgerichtet angeordnet. Ein grundsätzlich ähnlicher Aufbau ist in der Fig. 3 gezeigt, bei der jedoch das Rührerwellenpaar 9 mitsamt den Rührerwellen 10, 11 exzentrisch im Fermenterbehälter 3 in einem seitenwandnahen Bereich angeordnet ist. Die Deckenwand 6 dieses Fermenterbehälters 3 ist hier mittels einer zentralen und senkrecht ausgerichteten Mittelsäule 16 abgestützt. Ein weiterer Unterschied zur Ausführungsform nach Fig. 1 ist, dass hier auch die unteren Rührerwellenenden gelagert sind, und zwar im Bereich der Bodenwand 4. Ansonsten entspricht der Aufbau aber demjenigen der Fig. 1. In Fig. 4 ist ein Schnitt entlang der Linie B-B der Fig. 3 gezeigt.

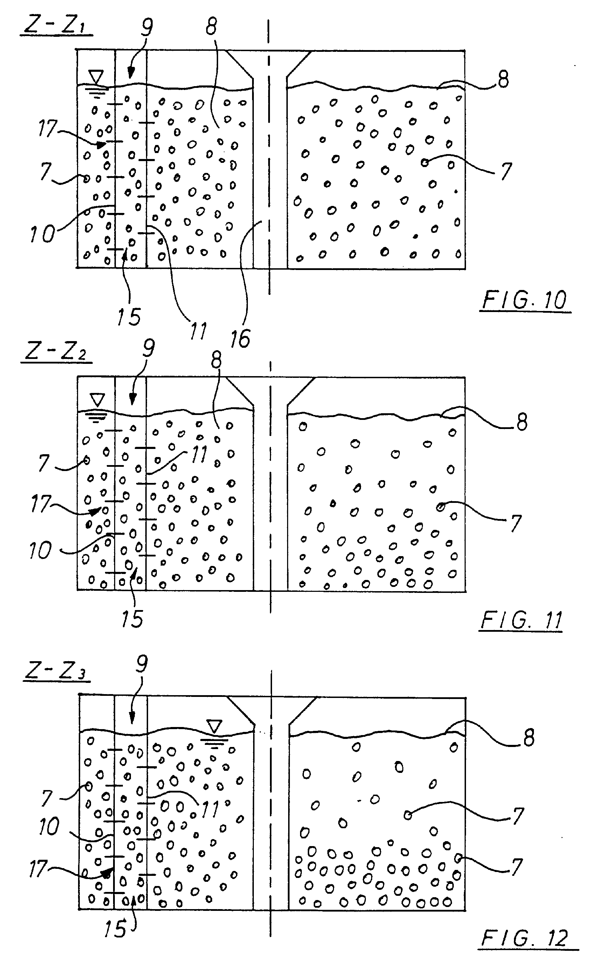

Die Funktionsweise der erfindungsgemäßen Rühreinrichtung 1 wird nachfolgend in Verbindung mit den Ausführungsformen der Figuren 1 bis 4 sowie unter Bezugnahme auf die Figuren 10 bis 12 näher erläutert:

Wie dies insbesondere aus den Figuren 2 und 4 ersichtlich ist, bilden die im Betriebszustand gegensinnig angetriebenen Rührerwellen 10, 11 in einem in den Figuren 2 und 4 schematisch dargestellten, strichliert umrandeten rührerwellenpaarnahen Fermenterflüssigkeitsbereich 17 als lokalen Durchmischungsbereich jeweils eine lokale, im Wesentlichen turbulente Rührerwellenströmung 18 um die jeweilige Rührerwelle 10, 11 herum aus. Dadurch wird in diesem Bereich, wie dies insbesondere aus der Fig. 10 ersichtlich wird, die eine schematische Schnittdarstellung entlang der Linie Z-Z1 der Fig. 4 zeigt, eine intensive lokale Vermischung und Durchmischung der Biomasse-Feststoffe 7 mit der Fermenterflüssigkeit 8 in eben diesem rührerwellenpaarnahen Fermenterflüssigkeitsbereich als lokalen Durchmischungsbereich 17 ausgebildet. Dadurch wird eine besonders gleichmäßige und regelmäßige Verteilung der Biomasse-Feststoffe 7 über die Fermenterflüssigkeit 8 in diesem lokalen Durchmischungsbereich 17 erzielt.

Zusätzlich zu dieser lokalen Rührerwellenströmung 18 bildet das Rührerwellenpaar 9 eine im Wesentlichen horizontale, gerichtete Umwälzströmung 19 im gesamten Volumen des Fermenterbehälters aus (Fig. 2, Fig. 4). Durch den gegensinnigen Antrieb der Rührerwellen 10, 11 bildet sich in Umwälzströmungsrichtung gesehen hinter dem Rührerwellenpaar 9 ein Überdruckbereich 20 und in Umwälzströmungsrichtung gesehen vor dem Rührerwellenpaar 9 ein Unterdruckbereich als Ansaugbereich 21 aus, der bezogen auf den Durchströmbereich 15 und die Rührerwellen 10, 11 zwischen den beiden Rührerwellen 10, 11 gegenüberliegend zum Überdruckbereich 20 angeordnet ist. Dadurch kann der sehr gut und intensiv mit Biomasse-Feststoffen 7 durchmischte Fermenterflüssigkeitsteil nach Durchströmen des Durchströmbereichs 15 vom Überdruckbereich 20 ausgehend in den rührerwellenpaarfernen Fermenterflüssigkeitsbereich wegströmen und anschließend wieder im Kreislauf zum Ansaugbereich 21 zurückströmen, wie dies in den Figuren 2 und 4 beispielhaft dargestellt ist.

Im Falle der Ausführung nach den Figuren 1 und 2, bei der das Rührerwellenpaar 9 in einem mittleren zentralen Fermenterbereich angeordnet ist, bildet sich eine horizontale Umwälzströmung aus, die sich nach dem Wegströmen vom Überdruckbereich 20 aufteilt in eine erste horizontale Umwälzteilströmung 22 und in eine entgegengesetzt gerichtete, zweite horizontale Umwälzteilströmung 23. Nach dem Wegströmen vom Überdruckbereich 20 strömt dieser gut durchmischte Flüssigkeitsteil in Richtung Fermenterbehälter-Seitenwand 5, an der sich die horizontale Umwälzströmung 19 in einem Gabelungsbereich 24 in die erste und zweite horizontale Umwälzteilströmung aufteilt. Anschließend strömen die beiden horizontalen Umwälzteilströmungen 22, 23 wieder im Kreislauf zum Ansaugbereich 21 zurück, wobei die beiden Umwälzteilströme 22, 23 in einem bezogen auf das Rührerwellenpaar 9 dem Gabelungsbereich 24 gegenüberliegenden Vereinigungsbereich 25 wieder aufeinanderprallen und sich vereinigen.

Im Gegensatz dazu wird bei einer Anordnung gemäß dem Ausführungsbeispiel der Figuren 3 und 4, d. h., der seitenwandnahen, exzentrischen Anordnung des Rührerwellenpaares 9 eine einzige gleichgerichtete Haupt-Umwälzströmung als horizontale Umwälzströmung 19 im Wesentlichen um die Mittelsäule 16 herum ausgebildet.

Die gute und gleichmäßige Verteilung der Biomasse 7 bezogen auf das gesamte Behältervolumen wird anhand der Fig. 10 bis 12, die unterschiedliche Schnittdarstellungen der Fig. 4 zeigen beispielhaft für die Ausführungsform der Fig. 3 und 4 erläutert.

Wie die Schnitte Z-Z1, Z-Z2 und Z-Z3 der Figuren 10, 11 und 12 zeigen, wird durch die horizontale Umwälzströmung 19 jeweils eine gleichmäßige Verteilung der Biomasse-Feststoffe 7 in der gesamten Fermenterflüssigkeit 8 erzielt. Für den hier beispielhaft gewählten Fall, dass es sich bei den Biomasse-Feststoffen 7 um Sinkstoffe handelt, die im Nichtbetrieb der Rühreinrichtung 1 Sinkschichten ausbilden würden, ist der Grad der Gleichmäßigkeit der Verteilung der Biomasse-Feststoffe 7 in der Fermenterflüssigkeit 8 nach dem Durchströmen des Durchströmbereichs 15 am höchsten und nimmt im Verlauf des Umwälzströmungsweges zwischen dem Überdruckbereich 20 und dem Ansaugbereich 21 in Abhängigkeit von der jeweiligen Biomasse und/oder der jeweiligen Fermenterflüssigkeit mehr oder weniger ab, wie dies in den Figuren 11 und 12 lediglich äußerst schematisch und prinzipiell dargestellt ist. So ist im Schnitt Z-Z3 die Entmischung bereits weiter fortgeschritten als im Schnitt Z-Z2. Da im lokalen Durchmischungsbereich 17 dann jedoch wieder eine intensive lokale Durchmischung der Biomasse-Feststoffe 7 mit der Fermenterflüssigkeit 8 erfolgt, ist eine insgesamt dauerhafte gleichmäßige und gute Verteilung der Biomasse-Feststoffe 7 in der Fermenterflüssigkeit 8 einstellbar, was sich besonders positiv auf den Wirkungsgrad des gesamten Fermentationsprozesses auswirkt.

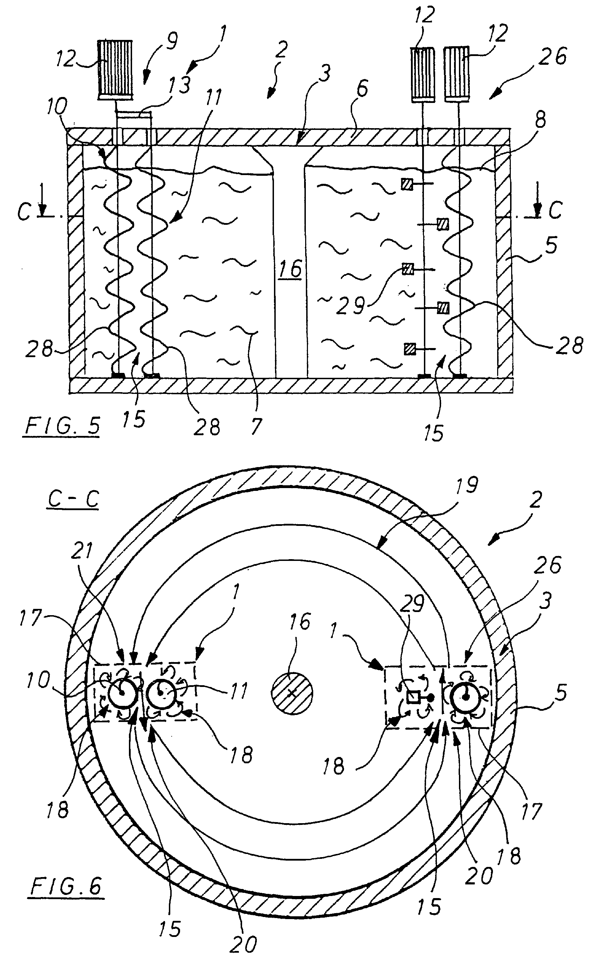

Übliche Fermenterbehälterdurchmesser liegen im Bereich von 10 bis 20 m, bevorzugt im Bereich von 13 bis 15 m, so dass dieser Durchmesser sehr groß ist gegenüber dem Durchmesser des Rührerwellenpaars 9, der z. B. im Bereich von etwa einem bis zwei Metern liegt. Dadurch kann es insbesondere bei sehr großen Innendurchmessern des Fermenterbehälters 3 erforderlich sein, wenigstens ein weiteres Rührerwellenpaar 9 vorzusehen, wie dies schematisch in den Figuren 5 und 6 dargestellt ist, wobei die Figur 6 einen schematischen Schnitt entlang der Linie C-C der Figur 5 zeigt. Hier ist in Umwälzströmungsrichtung gesehen beabstandet zum ersten exzentrischen Rührerwellenpaar 9 ein zweites exzentrisch angeordnetes Rührerwellenpaar 26 vorgesehen, das bezogen auf die in etwa senkrechte Mittelsäule 16 dem exzentrischen ersten Rührerwellenpaar 9 gegenüberliegt. Bei einem derartigen Aufbau wird die Umwälzströmung 19 beim Durchströmen der jeweiligen Durchströmbereiche 15 der Rührerwellenpaare 9, 10 nach einer dortigen lokalen intensiven Durchmischung wieder zur Verteilung der Biomasse-Feststoffe 7 in der gesamten Fermenterflüssigkeit 8, d. h. im gesamten Volumen des Fermenterbehälters 3 in Umwälzströmungsrichtung weiter gefördert, so dass über den gesamten Umwälzströmungsweg eine sehr gute und gleichmäßige Verteilung der Biomasse-Feststoffe 7 in der Fermenterflüssigkeit 8 erreicht werden kann. Das zweite oder jedes weitere Rührerwellenpaar wird somit in einem Bereich vorgesehen, wo der Grad der Entmischung bereits wieder so groß ist, dass eine gezielte Durchmischung erforderlich ist.

In der Fig. 3 ist schematisch und strichliert weiter ein liegendes Rührerwellenpaar 27 eingezeichnet, das hier in etwa waagrecht ausgerichtet lediglich beispielhaft exzentrisch im Fermenterbehälter angeordnet ist. Ein derartiges waagrecht liegendes Rührerwellenpaar 27 kann grundsätzlich alleine oder aber auch in Kombination mit mehreren waagrecht liegenden Rührerwellenpaaren oder aber auch in Kombination mit einem oder mehreren senkrechten Rührerwellenpaaren je nach den individuellen Erfordernissen und konkreten Gegebenheiten im Fermenterbehälter angeordnet werden. Dadurch ergeben sich die gleichen Ergebnisse bezüglich der Ausbildung einer lokalen Rührerwellenströmung 18 und einer horizontalen Umwälzströmung 19, wie dies zuvor in Verbindung mit den Figuren 1 bis 6 näher erläutert worden ist. Grundsätzlich kann es auch besonders vorteilhaft sein, z. B. in Verbindung mit einem Aufbau gemäß der Fig. 3 zwischen dem Rührerwellenpaar 9 und der Mittelsäule 16 ein weiteres Rührerwellenpaar einzusetzen, das quasi auf der gedachten Verbindungslinie zwischen Mittelsäule 16 und erstem Rührerwellenpaar 9 liegt, was hier aber nicht dargestellt ist. Auch dies kann z. B. insbesondere in Verbindung mit großen Fermenterbehältern vorteilhaft sein.

Die in den Figuren 1 bis 4 lediglich schematisch dargestellten Rührer 14 können zur Ausbildung einer eventuell gewünschten vertikalen Teilströmung gegebenenfalls z. B. schräg angestellt sein. Eine diesbezüglich besonders bevorzugte Ausführungsform ist in Fig. 5 gezeigt, bei der das Rührerwellenpaar 9 durch zwei Schnecken als Rührerwellen 10, 11 gebildet ist, deren Schneckenwendel 28 als Rührer fungieren, die z. B. je nach Antriebsrichtung der Schnecke auch eine überlagerte Vertikalteilströmung ausbilden können. Die Schneckenwendel 28 der Rührerwellen 10, 11 können in einer Doppelfunktion gleichzeitig z. B. wenigstens teilweise als Zerkleinerungseinrichtung, z. B. durch aufgeschweißte Schlagplatten oder dergleichen, ausgebildet sein, so dass die in den Durchströmbereich 15 gelangende Biomasse, insbesondere Biomasse-Feststoffe dort auch noch vorteilhaft zerkleinert werden können. Dies ist besonders vorteilhaft in Verbindung mit zwei Schnecken als Rührerwellen 10, 11 eines Rührerwellenpaares. Grundsätzlich ist jedoch auch eine Kombination von z. B. einer Schnecke als Rührerwelle mit einer Rührerwelle die Rührerpaddel 29 als Rührer aufweist möglich, wie dies beispielhaft für das zweite Rührerwellenpaar 26 der Fig. 5 gezeigt ist. Auch hier können die Rührerpaddel 29, die vorzugsweise angeschweißt sind, gegebenenfalls schräg angestellt werden für eine Ausbildung einer zusätzlichen überlagerten Vertikalteilströmungskomponente.

In der Fig. 5 ist ferner in Verbindung mit dem zweiten Rührerwellenpaar 26 beispielhaft gezeigt, dass für jede Rührerwelle des Rührerwellenpaares 26 auch ein separater Antrieb 12 vorgesehen sein kann, z. B. um unterschiedliche Antriebsgeschwindigkeiten der Rührerwellen zu bewerkstelligen.

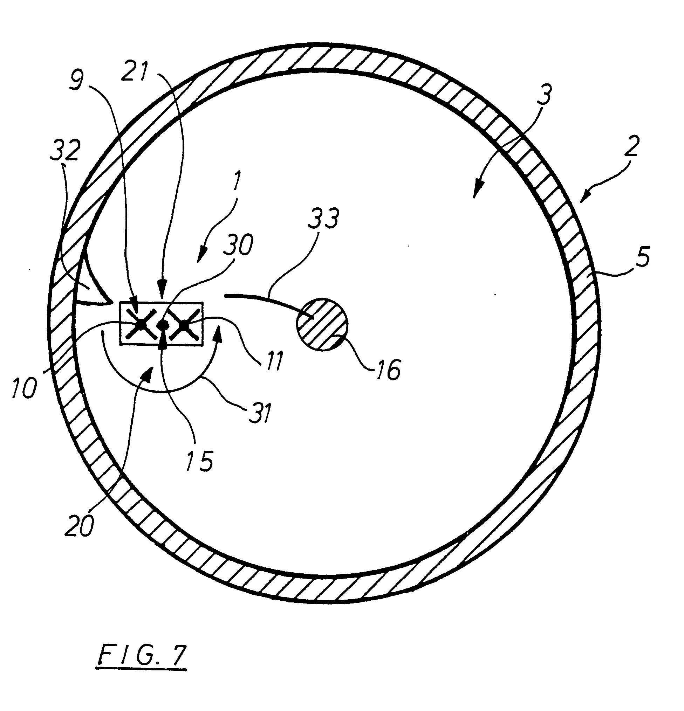

Äußerst schematisch und vom Prinzip her dargestellt ist in Fig. 7, dass das Rührerwellenpaar 9 auch so im Fermenterbehälterinnenraum des Fermenterbehälters 3 gelagert werden kann, dass dieses um eine in Rührerwellenlängsrichtung verlaufende Schwenkachse 30 verschwenkbar ist, wie dies durch den Pfeil 31 angezeigt ist. Vorteilhaft ist eine Festlegung in den unterschiedlichen Schwenkpositionen möglich.

In der Fig. 7 sind ferner Strömungsleitbleche 32, 33 beispielhaft vorgesehen, die in Verbindung mit einer gegen den Uhrzeigersinn gerichteten und hier nicht dargestellten horizontalen Umwälzströmung eine besonders vorteilhafte Zuführung dieser Umwälzströmung im Ansaugbereich 21 sicherstellen.

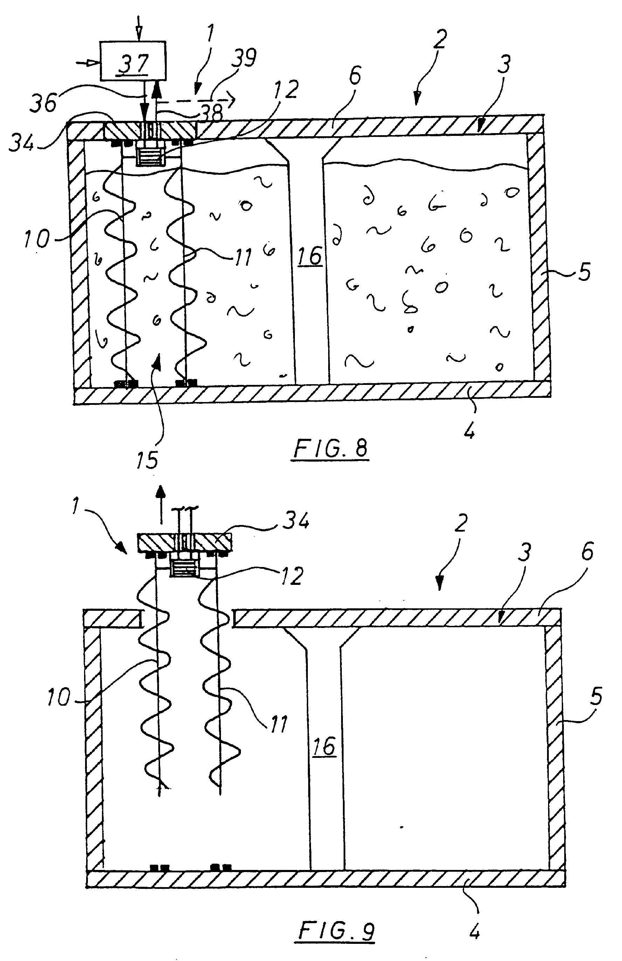

In den Figuren 8 und 9 ist eine weitere vorteilhafte Ausführungsvariante gezeigt, bei der das Rührerwellenpaar 9 als in den Fermenterbehälter 3 einsetzbares und herausnehmbares Rührerwellenmodul ausgebildet ist. Dazu weist das Rührerwellenmodul eine in die Deckenwand 6 einsetzbare Lagerplatte 34 auf, an der die Rührerwellen 10, 11 mit einem Rührerwellenende hier beispielhaft unmittelbar gelagert sind. Weiter ist hier auch der Antrieb 12 Bestandteil des Rührerwellenmoduls, der hier unterhalb der Lagerplatte 34 angeordnet ist. Der Antrieb 12 ist hier z. B. als Hydraulik-Motor oder Pneumatik-Motor ausgebildet, wobei das Medium entsprechend eine Hydraulikflüssigkeit beim Hydraulik-Motor oder Luft beim Pneumatik-Motor ist. Ein derartiger Antrieb mittels einem Arbeitsmedium weist eine Energieleitung 35 auf, die einerseits eine z. B. Schlauchleitung als Zuführleitung 36 für das Medium aufweist, die an eine Medium-Kompressionseinheit 37, z. B. einen Hydraulik-Kompressor oder einen Luft-Kompressor, angeschlossen ist und die dicht durch die Lagerplatte 34 als Bestandteil der Deckenwand 6 geführt ist. Weiter umfasst diese Energieleitung 35 auch eine z. B. Schlauchleitung als Abführleitung 38 für das Medium, die ebenfalls dicht durch die Lagerplatte 34 als Bestandteil der Deckenwand 6 aus dem Fermenterbehälter 3 herausgeführt ist. Die Abführleitung 38 ist im Falle eines Hydraulik-Motors als Rückleitung in einem geschlossenen HydraulikKreislauf an den Hydraulik-Kompressor angeschlossen, während sie im Falle eines Pneumatik-Motors als Abblasleitung auch in die Umgebung abblasen kann, wie dies in der Fig. 8 strichliert durch den Pfeil 39 dargestellt ist. Auch die Medium-Kompressionseinheit 37 kann selbstverständlich an der Lagerplatte 34 gelagert werden.

Wie aus den Figuren 8 und 9 ersichtlich ist, kann das gesamte Rührwellenmodul dann zur Montage bereits im vormontierten Zustand in den Fermenterbehälter einfach und schnell eingesetzt werden. Genau so gut kann aber auch eine Entnahme des Rührerwellenmoduls zu Wartungs- und/oder Reparaturarbeiten auf schnelle und einfache Weise erfolgen, wie dies in der Fig. 9 gezeigt ist.

## Patentansprüche

1. Rühreinrichtung für einen Fermenter einer Biogasanlage
mit einer Rührerwelle, die wenigstens einen Rührer aufweist, wobei die Rührerwelle mittels einem Rührerwellenantrieb antreibbar ist und wobei die Rührerwelle in eine in einem Fermenterbehälterinnenraum aufnehmbare und mit Biomasse versetzte Fermenterflüssigkeit eintauchbar ist zum Umwälzen der Fermenterflüssigkeit zur Verteilung der Biomasse in der Fermenterflüssigkeit,
**dadurch gekennzeichnet,**
**dass** zwei einander zugeordnete und im Betriebszustand gegensinnig angetriebene Rührerwellen (10,11) als Rührerwellenpaar (9; 26; 27) vorgesehen sind, die in etwa parallel zueinander ausgerichtet sind und die im in die Fermenterflüssigkeit (8) eingetauchten, angetriebenen Zustand eine lokale, im Wesentlichen turbulente Rührerwellenströmung (18) um die jeweilige Rührerwelle (10, 11) herum ausbilden für eine intensive lokale Vermischung der Biomasse (7) mit der Fermenterflüssigkeit (8) im rührerwellenpaarnahen Fermenterflüssigkeitsbereich (17), und
die im in die Fermenterflüssigkeit (8) eingetauchten Zustand ferner eine im Wesentlichen horizontale, gerichtete Umwälzströmung (19) im gesamten Volumen des Fermenterbehälters (3) ausbilden dergestalt, dass die im Wesentlichen horizontale Umwälzströmung (19) nach Durchströmen eines Durchströmbereiches (15) zwischen den beiden Rührerwellen (10, 11) von einem im Bereich der Rührerwellen (10, 11) ausgebildeten Überdruckbereich (20) ausgehend in den rührerwellenpaarfernen Fermenterflüssigkeitsbereich wegströmt und anschließend wieder im Kreislauf zu einem Unterdruckbereich als Ansaugbereich (21).

2. Rühreinrichtung nach Anspruch 1, **dadurch gekennzeichnet,**
**dass** die Rührerwellen (10, 11) beabstandet zur Fermenterbehälterwand (5), vorzugsweise in einem in etwa zentralen Fermenterbereich, in die Fermenterflüssigkeit (8) eintauchbar sind dergestalt,
**dass** sich zusätzlich zur lokalen Rührerwellenströmung (18) in der gesamten Fermenterflüssigkeit (8) eine Umwälzströmung (19) einstellt, die sich nach dem Wegströmen vom Überdruckbereich (20) aufteilt in eine erste horizontale Umwälzteilströmung (22) und in eine entgegengesetzt gerichtete zweite horizontale Umwälzteilströmung (23), die in einem Gabelungsbereich (24) voneinander weg in eine entgegengesetzte Richtung strömen und anschließend im Kreislauf wieder zum Ansaugbereich (21) zurückströmen, ggf. unter vorheriger Vereinigung der beiden Umwälzströme (22, 23) in einem Vereinigungsbereich (25).

3. Rühreinrichtung nach Anspruch 1, **dadurch gekennzeichnet,**
**dass** die Rührerwellen (10, 11) exzentrisch, vorzugsweise im fermenterbehälterwandnahen Fermenterbereich, in die Fermenterflüssigkeit (8) eintauchbar sind, vorzugsweise exzentrisch zu einer im zentralen Fermenterbereich in etwa senkrecht angeordneten Mittelsäule (16) des Fermenterbehälters (3), dergestalt,
**dass** sich zusätzlich zur lokalen Rührerwellenströmung (18) in der gesamten Fermenterflüssigkeit (8) im Wesentlichen eine einzige gleichgerichtete Haupt-Umwälzströmung (19) einstellt.

4. Rühreinrichtung nach einem der Ansprüche 1 bis 4, **dadurch gekennzeichnet, dass** das Rührerwellenpaar (9; 26; 27) bezogen auf den Fermenterbehälter (3) in etwa senkrecht oder waagrecht ausgerichtet in die Fermenterflüssigkeit (8) eintauchbar ist.

5. Rühreinrichtung nach einem der Ansprüche 1 bis 4, **dadurch gekennzeichnet,**
**dass** in Umwälzströmungsrichtung gesehen beabstandet zum ersten Rührerwellenpaar (9) wenigstens ein weiteres Rührerwellenpaar (26) vorgesehen ist, vorzugsweise ein zweites exzentrisch angeordnetes und bezogen auf eine in etwa senkrechte Mittelsäule (16) vorzugsweise dem exzentrischen ersten Rührerwellenpaar (9) gegenüberliegendes, Rührerwellenpaar (10) vorgesehen ist, dergestalt,
**dass** die Umwälzströmung (19) beim Durchströmen des Durchströmbereichs (15) des jeweiligen Rührerwellenpaares (9, 26; 27) nach einer dortigen lokalen intensiven Durchmischung wieder in Umwälzströmungsrichtung (19) weiterförderbar ist.

6. Rühreinrichtung nach einem der Ansprüche 1 bis 5, **dadurch gekennzeichnet, dass** das Rührerwellenpaar (9) so im Fermenterbehälterinnenraum lagerbar ist, dass dieses um eine in Rührerwellenlängsrichtung verlaufende Schwenkachse (30) verschwenkbar und vorzugsweise in unterschiedlichen Schwenkpositionen festlegbar ist.

7. Rühreinrichtung nach einem der Ansprüche 1 bis 6, **dadurch gekennzeichnet,**
**dass** jedes Rührerwellenpaar (9; 27) einen gemeinsamen Antrieb (12) für beide Rührerwellen (10, 11) aufweist, und
**dass** der Antrieb (12) eine der Rührerwellen (10) antreibt und ferner Mittel (13) vorgesehen sind, mit denen eine Antriebskraft auf die jeweils andere Rührerwelle (11) des Rührerwellenpaars (9; 27) übertragbar ist.

8. Rühreinrichtung nach einem der Ansprüche 1 bis 7, **dadurch gekennzeichnet, dass** der wenigstens eine Antrieb (12), vorzugsweise ein Elektromotor, außerhalb des Fermenterbehälters (3) angeordnet ist.

9. Rühreinrichtung nach einem der Ansprüche 1 bis 7, **dadurch gekennzeichnet,**
**dass** der wenigstens eine Antrieb (12) im Fermenterbehälter (3) anordenbar und mittels einem Arbeitsmedium antreibbar ist,
**dass** der Antrieb mit einer Energieleitung (35) verbunden ist,
**dass** die Energieleitung (35) einerseits eine Rohr- und/oder Schlauchleitung als Zuführleitung (36) für das Medium, die an eine Medium-Kompressionseinheit (37) angeschlossen ist und die dicht durch eine Behälterwand (6) zum Antrieb (12) geführt ist, und andererseits eine Rohrund/oder Schlauchleitung als Abführleitung (38) für das Medium, die dicht durch eine Behälterwand (6) aus dem Fermenterbehälter (3) herausgeführt ist, umfasst, und
**dass** die Medium-Kompressionseinheit (37) vorzugsweise elektrisch betreibbar ist und vorzugsweise außerhalb des Fermenterbehälters (3) angeordnet ist.

10. Rühreinrichtung nach Anspruch 9, **dadurch gekennzeichnet,**
**dass** der Antrieb (12) ein Hydraulik-Motor ist, wobei das Medium eine Hydraulikflüssigkeit ist und die Medium-Kompressionseinheit (37) ein Hydraulik-Kompressor ist, und
**dass** die Abführleitung (38) als Rückleitung in einem geschlossenen Hydraulikkreislauf an den Hydraulik-Kompressor angeschlossen ist.

11. Rühreinrichtung nach Anspruch 9, **dadurch gekennzeichnet,**
**dass** der Antrieb (12) ein Pneumatik-Motor ist, wobei das Medium Luft ist und die Medium-Kompressionseinheit (37) ein Luftkompressor ist, und
**dass** die Abführleitung (38) als Abblasleitung dicht aus dem Fermenterbehälter (3) geführt ist.

12. Rühreinrichtung nach einem der Ansprüche 1 bis 11, **dadurch gekennzeichnet, dass** die Rührerwellen (10, 11) jedes Rührerwellenpaares (9; 26; 27) mit unterschiedlichen Geschwindigkeiten und/oder in eine Vorwärts- oder Rückwärtsrichtung antreibbar sind.

13. Rühreinrichtung nach einem der Ansprüche 1 bis 12, **dadurch gekennzeichnet, dass** der Durchströmbereich (15) so ausgebildet ist, dass die Rührerwellen (10, 11) mit ihren Rührern einen Spaltabstand voneinander aufweisen oder dass die Rührerwellen (10, 11) mit ihren Rührern im Betrieb wenigstens teilweise ineinandergreifen.

14. Rühreinrichtung nach einem der Ansprüche 1 bis 13, **dadurch gekennzeichnet, dass** die Rührerwellen (10, 11) im im Fermenterbehälter (3) montierten Zustand jeweils nur mit einem Rührerwellenende im Bereich der Behälterwand (6) mittelbar oder unmittelbar gelagert sind und das andere gegenüberliegende Rührerwellenende jeweils frei in die Fermenterflüssigkeit (8) eingetaucht ist.

15. Rühreinrichtung nach einem der Ansprüche 1 bis 14, **dadurch gekennzeichnet, dass** das Rührerwellenpaar (9; 26; 27) als in den Fermenterbehälter (3) einsetzbares und herausnehmbares Rührerwellenmodul ausgebildet ist.

16. Rühreinrichtung nach Anspruch 15, **dadurch gekennzeichnet,**
**dass** das Rührerwellenmodul eine in die Behälterwand (6) einsetzbare Lagerplatte (34) aufweist, an der die Rührerwellen (10, 11) mit einem Rührerwellenende mittelbar oder unmittelbar gelagert sind, und
**dass** vorzugsweise auch der wenigstens eine Antrieb (12) Bestandteil des Rührerwellenmoduls ist und je nach Ausführungsart des Antriebs (12) bezogen auf den montierten Zustand des Rührerwellenmoduls wenigstens zum Teil oberhalb oder unterhalb an der Lagerplatte (34) angeordnet ist.

17. Rühreinrichtung nach einem der Ansprüche 1 bis 16, **dadurch gekennzeichnet, dass** im Strömungsbereich der Umwälzströmung (19) wenigstens ein Strömungsleitblech (32, 33) und/oder wenigstens ein Strömungsbrecher vorgesehen ist.

18. Rühreinrichtung nach einem der Ansprüche 1 bis 17, **dadurch gekennzeichnet, dass** wenigstens eine der Rührerwellen (10, 11) durch eine Schnecke mit Schneckenwendel (28) als Rührer gebildet ist.

19. Rühreinrichtung nach Anspruch 18, **dadurch gekennzeichnet, dass** die Schneckenwendel (28) der Rührerwellen (10, 11) jedes Rührerwellenpaars (9; 26; 27) in einer Doppelfunktion wenigstens teilweise als Zerkleinerungseinrichtung für die in den Durchströmbereich (15) gelangende Biomasse (7) ausgebildet sind.

20. Rühreinrichtung nach einem der Ansprüche 1 bis 17, **dadurch gekennzeichnet, dass** wenigstens eine der Rührerwellen dort angebrachte, vorzugsweise dort angeschweißte Paddel (29) und/oder Balken und/oder Scheiben (14) und/oder Propeller und/oder Gitter als Rührer aufweist, die ggf. so angestellt sind, vorzugsweise schräg angestellt sind, dass sich eine zusätzliche, überlagerte Vertikalteilströmung ausbildet.

21. Verfahren zur Verteilung von Biomasse in einer Fermenterflüssigkeit mit einer Rühreinrichtung, insbesondere einer Rühreinrichtung nach einem der Ansprüche 1 bis 20,
bei dem mittels einer wenigstens einen Rührer aufweisenden Rührerwelle Biomasse in einer in einem Fermenterbehälter aufgenommenen Fermenterflüssigkeit verteilt wird,
**dadurch gekennzeichnet,**
**dass** mittels einem Rührwellenpaar (9; 26; 27), das zwei im Betriebszustand gegenläufige und in etwa parallel zueinander ausgerichtete Rührerwellen (10, 11) aufweist, eine lokale, im Wesentlichen turbulente Rührerwellenströmung (18) um die jeweilige Rührerwelle (10, 11) herum ausgebildet wird zur intensiven lokalen Vermischung der Biomasse (7) mit der Fermenterflüssigkeit (8) im rührerwellennahen Fermenterflüssigkeitsbereich,
**dass** im Betrieb des Rührerwellenpaars (9; 26; 27) zusätzlich eine im Wesentlichen horizontale, gerichtete Umwälzströmung (19) im gesamten Volumen des Fermenterbehälters (3) ausgebildet wird dergestalt,
**dass** die Umwälzströmung (19) nach intensiver lokaler Durchmischung der Biomasse (7) mit der Fermenterflüssigkeit beim Durchströmen eines Durchströmbereichs (15) zwischen den Rührerwellen (10, 11) von einem im Bereich der Rührerwellen (10, 11) ausgebildeten Überdruckbereich (20) ausgehend in den rührwellenfernen Fermenterflüssigkeitsbereich wegströmt und anschließend wieder im Kreislauf zu einem im Bereich der Rührerwellen (10, 11) ausgebildeten Unterdruckbereich als Ansaugbereich (21), wobei der Grad der Gleichmäßigkeit der Verteilung der Biomasse (7) in der Fermenterflüssigkeit (8) nach dem Durchströmen des Durchströmbereiches (15) am höchsten ist und im Verlauf des Umwälzströmungsweges zwischen einem Überdruckbereich (20) und einem Ansaugbereich (21) in Abhängigkeit von der jeweiligen Biomasse (7) und/oder Fermenterflüssigkeit (8) abnimmt.
